# EUROPEAN PATENT APPLICATION

(11) **EP 3 370 202 A1**
(43) Date of publication of application: **05.09.2018**
(21) Application number: 17787864.2
(22) Date of filing: 06.04.2017
(51) Int. Cl.: G06Q 50/20

(54) **DATA GENERATION METHOD AND DEVICE, TERMINAL, SERVER, AND STORAGE MEDIUM**

(30) Priority: 30.06.2016 CN 201610505729
(71) Applicant: Ping An Technology (Shenzhen) Co., Ltd., Shenzhen, Guangdong 518000 (CN)
(72) Inventor: ZHENG, Jiaer, Shenzhen Guangdong 518000 (CN); XU, Liang, Shenzhen Guangdong 518000 (CN)
(74) Representative: Michalski Hüttermann & Partner Patentanwälte mbB
(86) International application number: PCT/CN2017/079598
(87) International publication number: WO 2018/000883

(57) **Abstract**

A method of generating data includes: obtaining data to be processed, and the data to be processed comprises a drug purchasing time of a time dimension, medical consultation information of at least one dimension besides the time dimension, and association relationship between the medical consultation information of different dimensions, in which each dimension comprises at least one piece of medical consultation information; obtaining a preset center point and a preset radius value of a circle, and generating the circle according to the center point and the radius value; selecting a first dimension from the at least one dimension, and forming one corresponding radius in a respective direction in the circle for each piece of the medical consultation information of the first dimension, wherein each piece of the medical consultation information is corresponding to one radial direction; obtaining the drug purchasing time of the time dimension associated with the medical consultation information of the first dimension according to the association relationship between the medical consultation information of different dimensions, and determining a corresponding position of the associated drug purchasing time of the time dimension on the radius corresponding to the medical consultation information of the first dimension; and generating a node corresponding to the associated drug purchasing time of the time dimension at the corresponding position to form a timeline abnormality monitoring graph.

## Description

### CROSS-REFERENCE TO RELATED APPLICATION

This application claims benefit of Chinese Patent Application No. 2016105057293 filed on June 30, 2016, and entitled "METHOD AND DEVICE OF GENERATING DATA", which is hereby incorporated herein by reference in its entirety.

### FIELD OF THE INVENTION

The present disclosure relates to the field of data processing technology, and particularly relates to a method, a device, a terminal, a server, and a storage medium of generating data.

### BACKGROUND OF THE INVENTION

With continuous improvement of social security system, some costs of drugs and some other costs during the medical consultation can be reduced when patients use social security cards. Therefore, phenomena of seeking improper profits by unauthorized use of social security card come thick and fast, such as making doctors prescribe a lot of expensive drugs and then selling the drugs to traffickers, insisting on being in hospital when not necessary in order to get costs reduced. Therefore, related institutions regularly analyze medical consultation information generated by using the social security card to obtain suspect medical consultation information, then further investigate the suspect medical consultation information to timely detect and prevent such improper means of seeking profits.

As amount of data is too large and people are not very sensitive to a simple number or text, the conventional way is to display the medical consultation information generated by using the social security card through basic types of charts, such as a line chart reflecting trend of money.

However, such conventional visualization charts only support the display of a single dimension at one moment, therefore the amount of information is small, and some of the key hidden information cannot be displayed.

### SUMMARY OF THE INVENTION

According to various embodiments disclosed herein by the application, a method, a device, a terminal, a server, and a storage medium of generating data are provided.

A method of generating data includes:
obtaining data to be processed, the data to be processed includes a drug purchasing time of a time dimension, medical consultation information of at least one dimension besides the time dimension, and association relationship between the medical consultation information of different dimensions, each dimension includes at least one piece of medical consultation information;
obtaining a preset center point and a preset radius value of a circle, and generating the circle according to the center point and the radius value; selecting a first dimension from the at least one dimension, and forming one corresponding radius in a respective direction in the circle for each piece of the medical consultation information of the first dimension; each piece of the medical consultation information is corresponding to one radial direction;
obtaining the drug purchasing time of the time dimension associated with the medical consultation information of the first dimension according to the association relationship between the medical consultation information of different dimensions, and determining a corresponding position of the associated drug purchasing time of the time dimension on the radius corresponding to the medical consultation information of the first dimension; and
generating a node corresponding to the associated drug purchasing time of the time dimension at the corresponding position to form a timeline abnormality monitoring graph.

A device of generating data includes:
an obtaining module configured to obtain data to be processed; the data to be processed includes drug purchasing time of a time dimension, medical consultation information of at least one dimension besides the time dimension, and association relationship between the medical consultation information of different dimensions; each dimension includes at least one piece of medical consultation information;
a radius forming module configured to obtain a preset center point and a preset radius value of a circle, and generate the circle according to the center point and the radius value; select a first dimension from the at least one dimension, and form one corresponding radius in a respective direction in the circle for each piece of the medical consultation information of the first dimension; each piece of the medical consultation information is corresponding to one radial direction;
a position determining module configured to obtain the drug purchasing time of the time dimension associated with the medical consultation information of the first dimension according to the association relationship between the medical consultation information of different dimensions, and determine a corresponding position of the associated drug purchasing time of the time dimension on the radius corresponding to the medical consultation information of the first dimension; and
a generating module configured to generate a node corresponding to the associated drug purchasing time of the time dimension at the corresponding position to form a timeline abnormality monitoring graph.

A terminal includes a processor, and a memory that stores instructions, which, when executed by the processor, cause the processor to perform the following steps:
obtaining data to be processed; the data to be processed includes a drug purchasing time of a time dimension, medical consultation information of at least one dimension besides the time dimension, and association relationship between the medical consultation information of different dimensions; each dimension includes at least one piece of medical consultation information;
obtaining a preset center point and a preset radius value of a circle, and generating the circle according to the center point and the radius value; selecting a first dimension from the at least one dimension, and forming one corresponding radius in a respective direction in the circle for each piece of the medical consultation information of the first dimension; each piece of the medical consultation information is corresponding to one radial direction;
obtaining the drug purchasing time of the time dimension associated with the medical consultation information of the first dimension according to the association relationship between the medical consultation information of different dimensions, and determining a corresponding position of the associated drug purchasing time of the time dimension on the radius corresponding to the medical consultation information of the first dimension; and
generating a node corresponding to the associated drug purchasing time of the time dimension at the corresponding position to form a timeline abnormality monitoring graph.

A server includes a processor, and a memory that stores instructions, which, when executed by the processor, cause the processor to perform the following steps:
obtaining data to be processed; the data to be processed includes a drug purchasing time of a time dimension, medical consultation information of at least one dimension besides the time dimension, and association relationship between the medical consultation information of different dimensions; each dimension includes at least one piece of medical consultation information;
obtaining a preset center point and a preset radius value of a circle, and generating the circle according to the center point and the radius value; selecting a first dimension from the at least one dimension, and forming one corresponding radius in a respective direction in the circle for each piece of the medical consultation information of the first dimension; each piece of the medical consultation information is corresponding to one radial direction;
obtaining the drug purchasing time of the time dimension associated with the medical consultation information of the first dimension according to the association relationship between the medical consultation information of different dimensions, and determining a corresponding position of the associated drug purchasing time of the time dimension on the radius corresponding to the medical consultation information of the first dimension; and
generating a node corresponding to the associated drug purchasing time of the time dimension at the corresponding position to form a timeline abnormality monitoring graph.

At least one non-transitory computer-readable storage medium that stores computer-executable instructions, which, when executed by at least one processor, cause the at least one processor to perform the following steps:
obtaining data to be processed; the data to be processed includes a drug purchasing time of a time dimension, medical consultation information of at least one dimension besides the time dimension, and association relationship between the medical consultation information of different dimensions; each dimension includes at least one piece of medical consultation information;
obtaining a preset center point and a preset radius value of a circle, and generating the circle according to the center point and the radius value; selecting a first dimension from the at least one dimension, and forming one corresponding radius in a respective direction in the circle for each piece of the medical consultation information of the first dimension; each piece of the medical consultation information is corresponding to one radial direction;
obtaining the drug purchasing time of the time dimension associated with the medical consultation information of the first dimension according to the association relationship between the medical consultation information of different dimensions, and determining a corresponding position of the associated drug purchasing time of the time dimension on the radius corresponding to the medical consultation information of the first dimension; and
generating a node corresponding to the associated drug purchasing time of the time dimension at the corresponding position to form a timeline abnormality monitoring graph.

The details of one or more embodiments of the disclosure are set forth in the accompanying drawings and the description below. Other features, objects, and advantages of the disclosure will become apparent from the description, the drawings, and the claims.

### BRIEF DESCRIPTION OF THE DRAWINGS

To illustrate the technical solutions according to the embodiments of the present disclosure or in the prior art more clearly, the accompanying drawings for describing the embodiments or the prior art are introduced briefly in the following. Apparently, the accompanying drawings in the following description are only some embodiments of the present disclosure, and persons of ordinary skill in the art can derive other drawings from the accompanying drawings without creative efforts.
FIG. 1 is a diagram of an application environment in which a method of generating data is operated according to an embodiment.
FIG. 2 is a block diagram of a terminal according to an embodiment.
FIG. 3 is a block diagram of a server according to an embodiment.
FIG. 4 is a flow chart of a method of generating data according to an embodiment.
FIG. 5 is a diagram showing composition of a timeline abnormality monitoring graph according to an embodiment.
FIG. 6 is a flow chart of a method of adding node tag information according to an embodiment.
FIG. 7 is a diagram showing composition of a timeline abnormality monitoring graph according to another embodiment.
FIG. 8 is a flow chart of a method of determining a corresponding position according to an embodiment.
FIG. 9 is a flow chart of a method of viewing node related information according to an embodiment.
FIG. 10 is a block diagram of a device of generating data according to an embodiment.
FIG. 11 is a block diagram of a device of generating data according to another embodiment.
FIG. 12 is a block diagram of a device of generating data according to yet another embodiment.

### DETAILED DESCRIPTION OF THE EMBODIMENTS

The objects, features, and advantages of the present disclosure will become more apparent by describing in detail embodiments thereof with reference to the accompanying drawings. It should be understood that these embodiments depicted herein are only used to illustrate the present disclosure rather than limit the present disclosure.

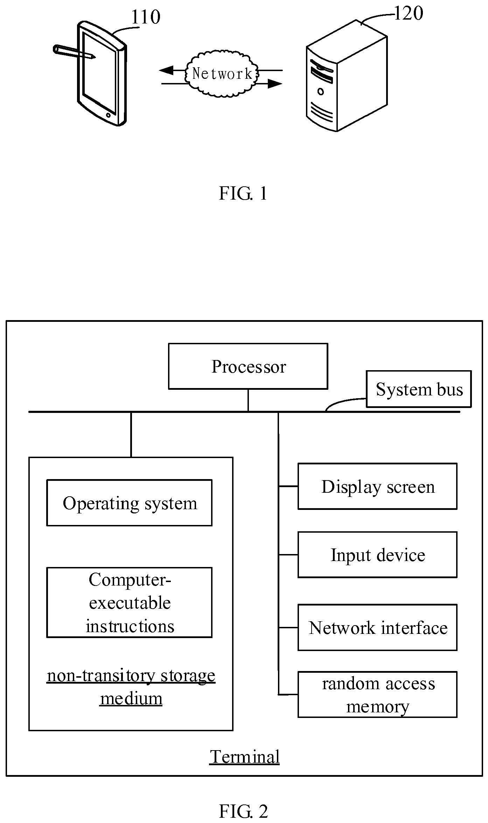

FIG. 1 is a diagram of an application environment in which a method of generating data is operated according to an embodiment. Referring to FIG. 1, a terminal 110 communicates with a server 120 through network.

In the application environment shown in FIG. 1, the terminal 110 can obtain data to be processed from the server 120 in response to instruction of obtaining the data to be processed operating on the terminal 110. The data to be processed includes a drug purchasing time of a time dimension, medical consultation information of at least one dimension besides the time dimension, and association relationship between the medical consultation information of different dimensions. Each dimension includes at least one piece of medical consultation information. The terminal 110 obtains a preset center point and a preset radius value of a circle from the local, and generates the circle according to the center point and the radius value; selects a first dimension from the at least one dimension, and forms one corresponding radius in a respective direction in the circle for each piece of the medical consultation information of the first dimension. Each piece of the medical consultation information is corresponding to one radial direction. Moreover, the terminal 110 obtains the drug purchasing time of the time dimension associated with the medical consultation information of the first dimension according to the association relationship between the medical consultation information of different dimensions, determines a corresponding position of the associated drug purchasing time of the time dimension on the radius corresponding to the medical consultation information of the first dimension, and generates a node corresponding to the associated drug purchasing time of the time dimension at the corresponding position to form a timeline abnormality monitoring graph. Furthermore, the terminal 110 can display the generated timeline abnormality monitoring graph on an interface of the terminal.

Alternatively, the terminal 110 sends a request of generating the timeline abnormality monitoring graph to the server 120, and the server 120 obtains the corresponding data to be processed from a database according to the request. The data to be processed includes a drug purchasing time of a time dimension, medical consultation information of at least one dimension besides the time dimension, and association relationship between the medical consultation information of different dimensions. Each dimension includes at least one piece of medical consultation information. The server 120 obtains a preset center point and a preset radius value of a circle, and generates the circle according to the center point and the radius value; selects a first dimension from the at least one dimension, and forms one corresponding radius in a respective direction in the circle for each piece of the medical consultation information of the first dimension. Each piece of the medical consultation information is corresponding to one radial direction. Moreover, the server 120 obtains the drug purchasing time of the time dimension associated with the medical consultation information of the first dimension according to the association relationship between the medical consultation information of different dimensions, and determines a corresponding position of the associated drug purchasing time of the time dimension on the radius corresponding to the medical consultation information of the first dimension, and generates a node corresponding to the associated drug purchasing time of the time dimension at the corresponding position to form a timeline abnormality monitoring graph. Furthermore, the terminal 120 can send the generated timeline abnormality monitoring graph to the terminal 110 so that the terminal 110 displays the timeline abnormality monitoring graph on the interface of the terminal.

In an embodiment, a terminal is provided on which an application can be installed. The terminal can be a mobile phone, a tablet computer, a notebook computer, a desktop computer, and so on. Referring to FIG. 2, the terminal includes a processor, a random access memory, a non-transitory storage medium, a network interface, a display screen, and an input device connected via a system bus. The processor is configured to provide calculation and control capabilities to support an operation of the entire terminal. The non-transitory storage medium of the terminal stores an operating system and computer-executable instructions. The computer-executable instructions can be executed by the processor to implement a method of generating data provided by the following embodiments. The random access memory of the terminal provides operating environment for the operating system and the computer-executable instructions stored in the non-transitory storage medium. The network interface is configured to be connected to a network for communication. The display screen of the terminal can be a liquid crystal display screen, an electronic ink display screen, and so on. In the present embodiment, the display screen can be used as an output device of the terminal for displaying various interfaces, for example, a timeline abnormality monitoring graph can be output and displayed. The input device can be a touch layer overlaid on the display screen; a button, a trackball, or a touch pad provided on the housing of the electronic device; an external keyboard, an external touch pad, or an external mouse; and so on. The input device is used by users to enter various control instructions, for example, it can be used by the user to input instruction of obtaining the data to be processed.

A person skilled in the art should understand that the structure illustrated in FIG. 2 is not intended to limit the terminal, more or less components than those shown in FIG. 2 may be included in the terminal, some components may be combined, or the components may be in another arrangement.

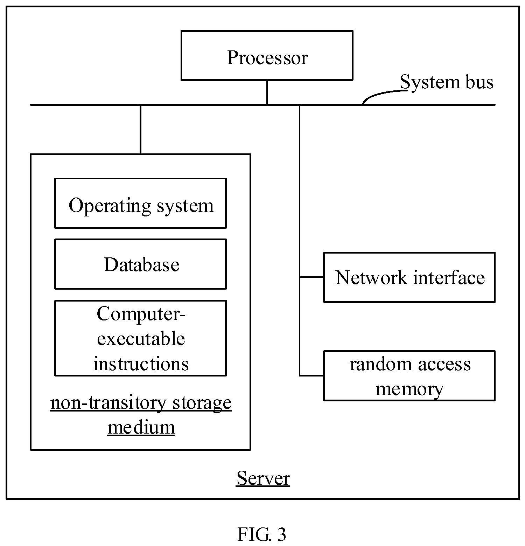

In an embodiment, a server is provided. Referring to FIG. 3, the server includes a processor, a non-transitory storage medium, a random access memory, and a network interface connected via a system bus. The processor is configured to provide calculation and control capabilities to support step of the entire server. The non-transitory storage medium of the server stores an operating system, a database, and computer-executable instructions. The database is used to store relevant data involved during the process of implementing a method of generating data, for example, a preset radius value can be stored. The computer-executable instructions can be executed by the processor to implement a method of generating data suitable for the server provided by the following embodiments. The random access memory of the server provides operating environment for the operating system and the computer-executable instructions stored in the non-transitory storage medium. The network interface is connected to network for communication. For example, in the present embodiment, the server can send a generated timeline abnormality graph to a terminal through the network interface. It should be understood that the server can be implemented as a server cluster which is formed by one server or multiple servers.

A person skilled in the art should understand that the structure illustrated in FIG. 3 is not intended to limit the server, more or less components than those shown in FIG. 3 may be included in the server, some components may be combined, or the components may be in another arrangement.

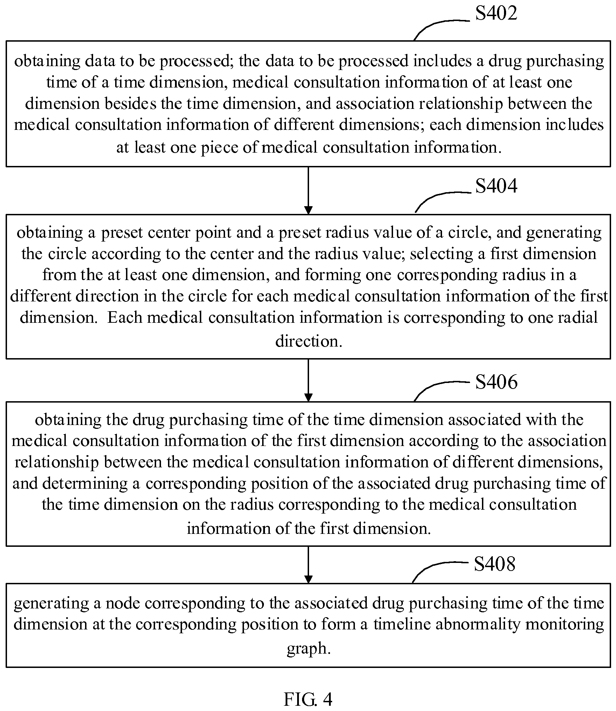

Referring to FIG. 4, in an embodiment, a method of generating data is provided, which is now applied to the terminal shown in FIG. 2 or the server shown in FIG. 3 as an example, and includes the following steps:
In step S402, data to be processed is obtained. The data to be processed includes a drug purchasing time of a time dimension, medical consultation information of at least one dimension besides the time dimension, and association relationship between the medical consultation information of different dimensions. Each dimension includes at least one piece of medical consultation information.

In the present embodiment, the obtained data to be processed includes the drug purchasing time of the time dimension, the medical consultation information of the at least one dimension besides the time dimension, and the association relationship between the medical consultation information of different dimensions. Each dimension includes at least one piece of medical consultation information. It should be understood that the drug purchasing time is the medical consultation information of the time dimension. The medical consultation information refers to a series of medical consultation related information generated during medical consultation process, such as information of a patient, a hospital, a doctor, time, a disease, a purchased drug, and a drug purchasing money amount during the medical consultation process.

Moreover, at least one dimension besides the time dimension includes a money amount dimension, a user dimension, a drug dimension, a hospital dimension, a medical project dimension, a doctor dimension, a disease dimension, and so on. Furthermore, the medical consultation information of the money amount dimension includes the drug purchasing money amount; the medical consultation information of the user dimension includes user information; the medical consultation information of the drug dimension includes drug information; the medical consultation information of the hospital dimension includes hospital information; the medical consultation information of the medical project dimension includes medical project information; the medical consultation information of the doctor dimension includes doctor information; the medical consultation information of the disease dimension includes disease information.

The association relationship between the medical consultation information of different dimensions refers to the interrelated association relationship between the medical consultation information of different dimensions during the medical consultation process. For example, if a user S purchases a drug P with a money amount M at time T, then the user S of the user dimension has an association relationship with the time T of the time dimension, the money amount M of the money amount dimension, and the drug P of the drug dimension. The drug P of the drug dimension has an association relationship with the time T of the time dimension, and the money amount M of the money amount dimension, and so on.

It should be understood that the definition of the association relationship can be configured according to actual demand, and there is no limitation that the association relationship exists between the medical consultation information of which specific dimensions.

In step S404, a preset center point and a preset radius value of a circle are obtained, and the circle is generated according to the center point and the radius value; a first dimension is selected from the at least one dimension, and one corresponding radius is formed in different directions in the circle for each piece of the medical consultation information of the first dimension. Each piece of the medical consultation information is corresponding to one radial direction.

In the present embodiment, the center point and the radius value are preset. The circle is generated according to the preset center point and the preset radius value, such that a basic configuration of the circle is formed.

A first dimension is selected from the at least one dimension. The first dimension can be the drug dimension, the user dimension, or other dimension. The first dimension can be configured according to actual composition requirements, and embodiments of the present disclosure are not limited thereto.

It should be understood that when there is only one dimension besides the time dimension, the selected first dimension is the only one dimension. When there are multiple dimensions besides the time dimension, the selected first dimension is one of the dimensions.

Furthermore, one corresponding radius is formed in different directions in the circle for each piece of the medical consultation information of the first dimension. Each piece of the medical consultation information is corresponding to one radial direction. Specifically, the radii of different directions can be randomly formed at any angle, or an angle range of the radius corresponding to the medical consultation information can be determined according to a type of the medical consultation information, and then the radius of the medical consultation information can be formed within the angle range of the radius. Alternatively, each piece of the medical consultation information is arranged according to preset rule, and the radius in a respective direction for each piece of the medical consultation information is sequentially formed according to preset angle interval threshold of the radius. The embodiments of the present disclosure have no limitation to how to form one corresponding radius in a respective direction for each piece of the medical consultation information of the first dimension, as long as it is satisfied that each of the medical consultation information is corresponding to one radial direction.

In step S406, according to the association relationship between the medical consultation information of different dimensions, the drug purchasing time of the time dimension associated with the medical consultation information of the first dimension is obtained, and a corresponding position of the associated drug purchasing time of the time dimension is determined on the radius corresponding to the medical consultation information of the first dimension.

In step S408, a node corresponding to the associated drug purchasing time of the time dimension is generated at the corresponding position to form a timeline abnormality monitoring graph.

In the present embodiment, the drug purchasing time of the time dimension associated with the medical consultation information of the first dimension is obtained according to the association relationship between the medical consultation information of different dimensions of the data to be processed. It should be understood that the minimum unit of the drug purchasing time of the time dimension can be determined on demand, and the drug purchasing time can be accurately measured in days, hours, minutes, seconds, and even smaller time units.

Moreover, a corresponding position of the associated drug purchasing time of the time dimension is determined on the radius corresponding to the medical consultation information of the first dimension.

Specifically, an earliest drug purchasing time of the time dimension is preset to be corresponding to a start point of the radius, and a latest drug purchasing time is preset to be corresponding to an end point of the radius. By determining where the associated drug purchasing time of the time dimension located between the earliest drug purchasing time and the latest drug purchasing time, the corresponding position of the associated drug purchasing time of the time dimension on the corresponding radius is determined.

Alternatively, the earliest drug purchasing time of the time dimension is preset to be corresponding to a start point of a certain segment of the radius, and the latest drug purchasing time is preset to be corresponding to an end point of the segment of the radius. By determining where the associated drug purchasing time of the time dimension located between the earliest drug purchasing time and the latest drug purchasing time, the corresponding position of the associated drug purchasing time of the time dimension is determined on the corresponding radius. The embodiment of the present disclosure have no limitation thereto, as long as the corresponding position of the associated drug purchasing time of the time dimension can be determined on the radius corresponding to the medical consultation information of the first dimension.

Furthermore, a node corresponding to the associated drug purchasing time of the time dimension is generated on the corresponding position of the associated drug purchasing time of the time dimension on the radius corresponding to the medical consultation information of the first dimension, and the timeline abnormality monitoring graph is formed.

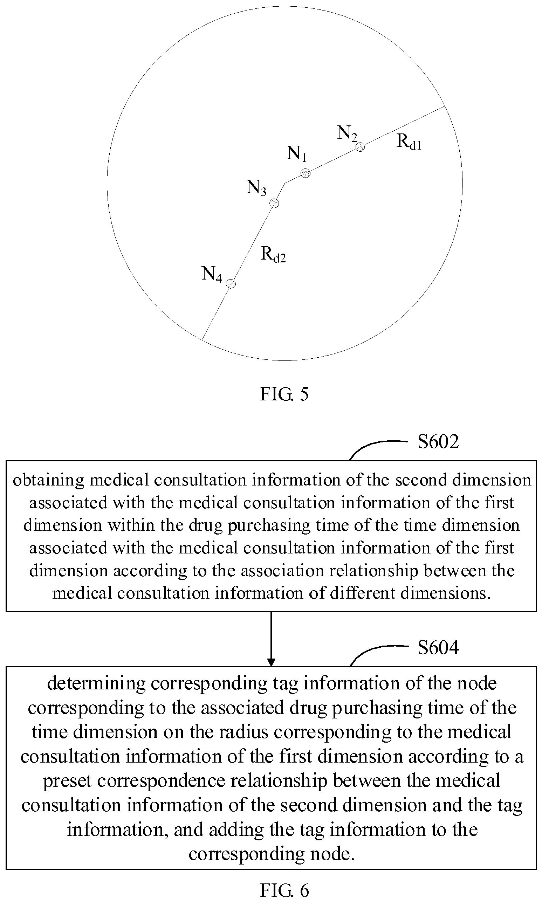

For example, the first dimension is the drug dimension which includes drug P₁, P₂; the drug P₁ is associated with the drug purchasing time T₁ and T₂, and the drug P₂ is associated with the drug purchasing time T₁ and T₃, then the corresponding timeline abnormality monitoring graph is shown as FIG. 5. Radii R_{d1}, R_{d2} in different directions in FIG. 5 correspond to the different drug P₁, P₂, respectively. Node N₁ is a node corresponding to the time T₁ on the radius R_{d1}, which is corresponding to the associated drug P₁; node N₂ is a node corresponding to the time T₂ on the radius R_{d2}, which is corresponding to the associated drug P₂; node N₃ is a node corresponding to the time T₁ on the radius R_{d2}, which is corresponding to the associated drug P₂; node N₄ is a node corresponding to the time T₃ on the radius R_{d2}, which is corresponding to the associated drug P₂. It should be understood that the actual timeline abnormality monitoring graph does not include the label shown in FIG. 5.

In the present embodiment, the circle is generated according to the preset center point and the preset radius value. The first dimension is selected from the at least one dimension, and one corresponding radius in a respective direction in the circle for each piece of the medical consultation information of the first dimension is formed. The drug purchasing time of the time dimension associated with the medical consultation information of the first dimension is obtained, and the corresponding position of the associated drug purchasing time of the time dimension on the radius corresponding to the medical consultation information of the first dimension is determined. The node corresponding to the associated drug purchasing time of the time dimension at the corresponding position is generated to form a timeline abnormality monitoring graph. Therefore, reflecting the drug purchasing time of the time dimension associated with the medical consultation information of the first dimension on the radius corresponding to the medical consultation information of the first dimension is achieved, which is equivalent to achieve the integration of the time dimension and the first dimension. Thus, rule of drug purchasing is reflected more accurately, and amount of information reflected on the graphics is increased.

In addition, a circle is used as a basic configuration and different medical consultation information of the first dimension is reflected by different radial directions. Since there is many situations how an angle of a circle can be divided and theoretically an angle can be divided infinitely, it is possible to view more medical consultation information of the first dimension at the same time, and the amount of information reflected on the graphics is further increased.

Furthermore, the timeline abnormality monitoring graph, which reflects multiple dimensions and large amount of information, can further reflect implied rule of drug purchasing, thus speed of finding suspect medical consultation information is improved.

Referring to FIG. 6, in an embodiment, the at least one dimension further includes a second dimension. The method further includes a step of adding node tag information, which specifically includes the following steps:
In step S602, according to the association relationship between the medical consultation information of different dimensions, medical consultation information of the second dimension associated with the medical consultation information of the first dimension within the drug purchasing time of the time dimension associated with the medical consultation information of the first dimension is obtained.

In the present embodiment, the at least one dimension further includes a second dimension. It should be noted that the "first" and "second" are only to distinguish between different dimensions, i.e., the second dimension is a dimension different from the first dimension, and are not intended to limit other aspects. In an embodiment, the first dimension includes a drug dimension, and the second dimension includes a user dimension; or the first dimension includes a user dimension, and the second dimension includes a drug dimension.

Furthermore, according to the association relationship between the medical consultation information of different dimensions, medical consultation information of the second dimension associated with the medical consultation information of the first dimension within the drug purchasing time of the time dimension associated with the medical consultation information of the first dimension is obtained.

In step S604, according to a preset correspondence relationship between the medical consultation information of the second dimension and the tag information, corresponding tag information of the node corresponding to the associated drug purchasing time of the time dimension on the radius corresponding to the medical consultation information of the first dimension is determined, then the tag information is added to the corresponding node.

In the present embodiment, correspondence relationship between the medical consultation information of the second dimension and the tag information is preset, and according to the correspondence relationship, corresponding tag information of the node corresponding to the drug purchasing time of the time dimension associated with the medical consultation information of the first dimension on the radius corresponding to the medical consultation information of the first dimension is determined.

It is can be understood that in step S602, medical consultation information of the second dimension associated with the medical consultation information of the first dimension within the drug purchasing time of the time dimension associated with the medical consultation information of the first dimension is obtained, and there is the node corresponding to the drug purchasing time of the time dimension associated with the medical consultation information of the first dimension on the radius corresponding to the medical consultation information of the first dimension, such that corresponding tag information of the node corresponding to the associated drug purchasing time of the time dimension on the radius corresponding to the medical consultation information of the first dimension can be determined according to the correspondence relationship between the medical consultation information of the second dimension and the tag information. The tag information can be color information, shape information, and so on.

Furthermore, the determined tag information is added to the corresponding node.

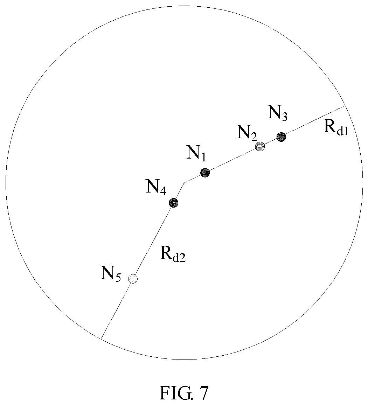

For example, the first dimension is the drug dimension which includes drug P₁, P₂, and the second dimension is the user dimension. The drug P₁ is associated with the drug purchasing time January 1, 2016, January 3, 2016, and January 4, 2016, respectively; the drug P₂ is associated with the drug purchasing time January 1, 2016 and January 4, 2016, respectively. The person who purchased the drug P₁ on January 1, 2016 is user S₁ (i.e., within the drug purchasing time January 1, 2016, the user of the user dimension who is associated with the drug P₁ of the drug dimension is user S₁), and the user S₁ also purchased the drug P₂. The person who purchased the drug P₁ on January 3, 2016 is the user S₂. The person who purchased the drug P₁ on January 4, 2016 is the user S₁. The person who purchased the drug P₂ on January 4, 2016 is the user S₃. The tag information corresponding to the user S₁ is represented by color 1; the tag information corresponding to the user S₂ is represented by color 2; and the tag information corresponding to the user S₃ is represented by color 3. The corresponding timeline abnormality monitoring graph is shown in FIG. 7. The radii R_{d1}, R_{d2} in different directions in FIG. 7 are corresponding to the drug P₁, P₂, respectively. The node N₁ is a node of the drug purchasing time January 1, 2016 on the radius R_{d1} corresponding to the drug P₁, which is associated with the drug purchasing time January 1, 2016; the node N₂ is a node of the drug purchasing time January 3, 2016 on the radius R_{d1} corresponding to the drug P₁, which is associated with the drug purchasing time January 3, 2016; the node N₃ is a node of the drug purchasing time January 4, 2016 on the radius R_{d1} corresponding to the drug P₁, which is associated with the drug purchasing time January 4, 2016; the node N₄ is a node of the drug purchasing time January 1, 2016 on the radius R_{d2} corresponding to the drug P₂, which is associated with the drug purchasing time January 1, 2016; the node N₅ is a node of the drug purchasing time January 4, 2016 on the radius R_{d2} corresponding to the drug P₂, which is associated with the drug purchasing time January 4, 2016. Furthermore, according to that the tag information corresponding to the user S₁ is represented by color 1, the tag information corresponding to the user S₂ is represented by color 2, and the tag information corresponding to the user S₃ is represented by color 3, on the radius R_{d1} which the drug P₁ is corresponding to, the nodes N₁, N₂, and N₃ of the drug purchasing time January 1, 2016, January 3, 2016, and January 4, 2016 are marked with color 1, color 2, and color 1, respectively; on the radius R_{d2} which the drug P₂ is corresponding to, the nodes N₄ and N₅ of the drug purchasing time January 1, 2016 and January 4, 2016 are marked with color 1 and color 3, respectively.

In the present embodiment, within the drug purchasing time of the time dimension associated with the medical consultation information of the first dimension, according to the tag information which the second dimension information associated with the medical consultation information of the first dimension is corresponding to, the corresponding tag information is added to the node of the drug purchasing time on the radius corresponding to the medical consultation information of the first dimension. Therefore, the integration of the three dimensions: the time dimension, the first dimension, and the second dimension is achieved. And the amount of information reflected on the graphics is further increased.

In addition, the timeline abnormality monitoring graph, which reflects multiple dimensions and large amount of information, can further reflect some key implied information, thus speed of finding suspect medical consultation information is improved.

In an embodiment, the medical consultation information of the at least one dimension includes a drug purchasing money amount of a money amount dimension. The method further includes a step of adjusting a size of a node, which specifically includes the following steps: obtaining the drug purchasing money amount of the money amount dimension associated with the medical consultation information of the first dimension within the drug purchasing time of the time dimension associated with the medical consultation information of the first dimension according to the association relationship between the medical consultation information of different dimensions; and adjusting the size of the node corresponding to the associated drug purchasing time of the time dimension on the radius corresponding to the medical consultation information of the first dimension according to the drug purchasing money amount.

In the present embodiment, the at least one dimension further includes the money amount dimension, the medical consultation information of the at least one dimension includes the drug purchasing money amount of the money amount dimension.

Furthermore, according to the association relationship between the medical consultation information of different dimensions, the drug purchasing money amount of the money amount dimension associated with the medical consultation information of the first dimension within the drug purchasing time of the time dimension associated with the medical consultation information of the first dimension is obtained. The size of the node corresponding to the associated drug purchasing time of the time dimension on the radius corresponding to the medical consultation information of the first dimension is adjusted according to the associated drug purchasing money amount.

It should be understood that it is the drug purchasing money amount associated with the medical consultation information of the first dimension within the drug purchasing time of the time dimension associated with the medical consultation information of the first dimension that to be obtained. For example, if the first dimension is the drug dimension, the medical consultation information of the first dimension is the drug P₁ and the drug P₂ of the drug dimension. The drug purchasing time T of the time dimension is corresponding to the drug P₁, and the drug purchasing time T of the time dimension is also corresponding to the drug P₂. Within time T, money amount M₁ is spent for purchasing the drug P₁, and money amount M₂ is spent for purchasing the drug P₂. The size of the node N₁ of the drug purchasing time T on the radius corresponding to the drug P₁ is adjusted according to money amount M₁, and the size of the node N₂ of the drug purchasing time T on the radius corresponding to the drug P₂ is adjusted according to money amount M₂.

Specifically, an interval of money amount corresponding to the associated drug purchasing money amount can be determined according to the preset correspondence relationship between the size of the node and the interval of drug purchasing money amount, then the size of the node corresponding to the interval of money amount is determined, thus the size of the node can be adjusted. Alternatively, a proportional relationship between the drug purchasing money amount and the size of the node is preset, and the size of the node is adjusted according to the proportional relationship. Moreover, the proportional relationship can be a preset constant ratio, or a dynamically variable formula of ratio.

Furthermore, correspondence relationship between the interval of money amount and the size of the node, or the proportional relationship between the drug purchasing money amount and the size of the node can be set up for each piece of the medical consultation information of the first dimension. For example, when the first dimension is the drug dimension, correspondence relationship between the interval of money amount and the size of the node can be set up for each drug information. Embodiments of the present disclosure have no limitation thereto. As long as the size of the node corresponding to the associated drug purchasing time of the time dimension can be adjusted according to the associated drug purchasing money amount.

In the present embodiment, within the drug purchasing time of the time dimension associated with the medical consultation information of the first dimension, according to the drug purchasing money amount of the money amount dimension associated with the medical consultation information of the first dimension, the size of the node of the drug purchasing time on the radius corresponding to the medical consultation information of the first dimension is adjusted. Therefore, the integration of the three dimensions: the time dimension, the first dimension, and the money amount dimension is achieved. And the amount of information reflected on the graphics is further increased.

In addition, the timeline abnormality monitoring graph, which reflects multiple dimensions and large amount of information, can further reflect some key implied information, thus speed of finding suspect medical consultation information is improved.

In other embodiments, the step of adding node tag information and the step of adjusting the size of the node can be combined, i.e., the step of adjusting the size of the node can performed after performing the step of adding node tag information; or the step of adding node tag information can be performed after performing the step of adjusting the size of the node. In the present embodiments, the step of adding node tag information and the step of adjusting the size of the node is combined. Therefore, the integration of the four dimensions: the time dimension, the first dimension, the second dimension, and the money amount dimension is achieved. The amount of information reflected on the graphics is further increased. In addition, some key implied information can be further reflect, thus speed of finding suspect medical consultation information is improved.

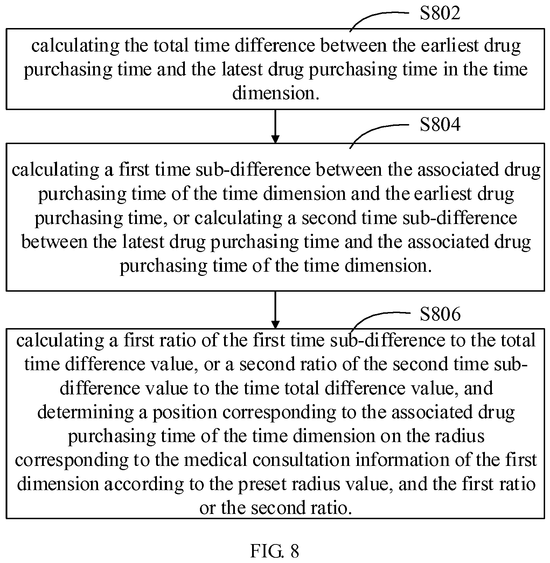

Referring to FIG. 8, in an embodiment, the step of determining a corresponding position of the associated medical consultation information of the time dimension on the radius corresponding to the medical consultation information of the first dimension (which is called "step of determining corresponding position" for short) includes the following steps:
In step S802, a total time difference between an earliest drug purchasing time and a latest drug purchasing time of the time dimension is calculated.
In step S804, a first time sub-difference between the associated drug purchasing time of the time dimension and the earliest drug purchasing time is calculated, or a second time sub-difference between the latest drug purchasing time and the associated drug purchasing time of the time dimension is calculated.

In the present embodiment, the time dimension includes at least one drug purchasing time. The total time difference between the earliest drug purchasing time and the latest drug purchasing time of the time dimension is calculated.

In addition, the first time sub-difference between the drug purchasing time of the time dimension associated with the medical consultation information of the first dimension and the earliest drug purchasing time is calculated, or the second time sub-difference between the latest drug purchasing time and the drug purchasing time of the time dimension associated with the medical consultation information of the first dimension is calculated.

In step S806, a first ratio of the first time sub-difference to the total time difference, or a second ratio of the second time sub-difference to the total time difference is calculated, and a position corresponding to the associated drug purchasing time of the time dimension on the radius corresponding to the medical consultation information of the first dimension is determined according to the preset radius value, and the first ratio or the second ratio.

Moreover, the first ratio of the first time sub-difference to the total time difference is calculated, or the second ratio of the second time sub-difference to the total time difference is calculated. The position corresponding to the associated drug purchasing time of the time dimension on the radius corresponding to the medical consultation information of the first dimension is determined according to the preset radius value, and the first ratio or the second ratio.

Specifically, a distance value can be obtained according to a product of the preset radius value and the first ratio or the second ratio, or the distance value can be obtained according to a product of the preset radius value, a distance adjusting factor, and the first ratio or the second ratio. The distance adjusting factor can be a preset constant, or a value that varies according to the differences between each associated drug purchasing time of the time dimension. Embodiments of the present disclosure have no limitation thereto.

Furthermore, a preset position corresponding to the earliest drug purchasing time on the radius is obtained. The preset position is a preset start point, and the position of the associated drug purchasing time of the time dimension on the radius corresponding to the medical consultation information of the first dimension located at the position which is one distance away from the preset start point. The preset start point can be the center point, or can be start point on the radius determined according to other rules.

For example, the earliest drug purchasing time is January 1, 2016, and the latest drug purchasing time is January 31, 2016. The preset radius value is R, and the preset start point is the center point. The associated medical consultation information of the time dimension, i.e., the drug purchasing time, is January 1, 2016, then the corresponding position of the associated medical consultation information of the time dimension on the radius is the position which is 12/31 ×R away from the center point.

In an embodiment, forming one corresponding radius in a respective direction in the circle for each piece of the medical consultation information of the first dimension includes the steps of: obtaining a preset type corresponding to each piece of the medical consultation information of the first dimension; obtaining an angle range of the radius corresponding to each piece of the medical consultation information of the first dimension according to a correspondence relationship between the preset type and the angle range of the radius; and forming a radius in a respective direction corresponding to each piece of the medical consultation information of the first dimension, which is corresponding to the angle range, within the obtained angle range in the circle.

In the present embodiment, the preset type corresponding to each piece of the medical consultation information of the first dimension is obtained, for example, when the first dimension is the drug dimension, the type corresponding to each drug information of the drug dimension is preset, and drug type can include a type of drugs for men, a type of drugs or women, and a type of generic drugs. In addition, when the first dimension is the drug dimension, the type of drug information can be preset according to users of drugs, the control of the amount of drugs, and restrictions on the use of drugs, and so on. It should be noted that the type of the medical consultation information of the first dimension can be configured according to actual demand.

Moreover, a correspondence relationship between the preset type and the angle range of the radius is preset, and according to the correspondence relationship, an angle range of the radius corresponding to each piece of the medical consultation information of the first dimension is obtained. Furthermore, within the obtained angle range of the radius in the circle, a radius in a respective direction corresponding to each piece of the medical consultation information of the first dimension, which is corresponding to the angle range of the radius, is formed.

For example, the first dimension is the drug dimension which includes the drug P₁ and the drug P₂. The preset type corresponding to the drug P₁ is the drugs for men, the preset type corresponding to the drug P₂ is the drugs for women. The angle range of the radius corresponding to drugs for men preset to be 0-120 degrees, the angle range of the radius corresponding to drugs for women preset to be 121-240 degrees, and the angle range of the radius corresponding to generic drugs preset to be 241-360 degrees. Then, within the angle range of the radius of 0-120 degrees, a radius corresponding to drug P₁ is formed; within the angle range of the radius of 121-240 degrees, a radius corresponding to drug P₂ is formed. At this point, if within the angle range of the radius corresponding to drugs for women some drugs are purchased in large quantities, and the purchaser is a male user, then the male user can be determined as a suspected user.

In the present embodiment, according to the type corresponding to the medical consultation information of the first dimension, the angle range of the radius corresponding to each piece of the medical consultation information of the first dimension is formed, then the corresponding radius of the medical consultation information can be formed within the angle range of the radius. Therefore, dimension of types of medical consultation information can be further reflect on the timeline abnormality monitoring graph, thus suspect medical consultation information can be found more quickly.

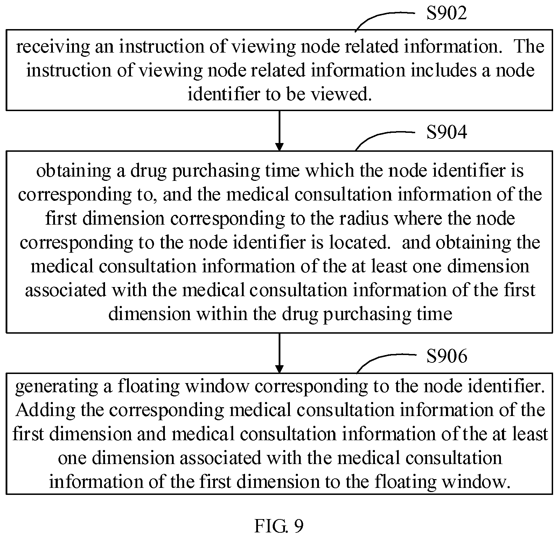

Referring to FIG. 9, in an embodiment, the method further includes a step of viewing node related information, which specifically includes the following steps:
In step S902, an instruction of viewing node related information is received. The instruction of viewing node related information includes a node identifier to be viewed.
In step S904, a drug purchasing time which the node identifier is corresponding to, and the medical consultation information of the first dimension corresponding to the radius where the node corresponding to the node identifier is located are obtained. The medical consultation information of the at least one dimension associated with the medical consultation information of the first dimension within the drug purchasing time is obtained.

Specifically, the receiving node association information viewing instruction includes a node identifier to be viewed in the association information viewing instruction. As mentioned above, the node is a node mark of the drug purchasing time of the time dimension at a corresponding position on the radius which the medical consultation information of the first dimension is corresponding to. Therefore, the node has a correspondence relationship with the drug purchasing time; the radius has a correspondence relationship with the medical consultation information of the first dimension. According to aforementioned correspondence relationships, the drug purchasing time which the node identifier is corresponding to can be obtained, and the medical consultation information of the first dimension corresponding to the radius where the node corresponding to the node identifier is located is obtained.

Moreover, according to the association relationship between the medical consultation information of different dimensions, the medical consultation information of the at least one dimension associated with the medical consultation information of the first dimension within the drug purchasing time is obtained. For example, when the first dimension is the drug dimension, user information of the user dimension, drug purchasing money amount of the money amount dimension, hospital information of the hospital dimension; doctor information of the doctor dimension; medical project information of the medical project dimension; disease information of the disease dimension, and so on associated with the drug information of the drug dimension within the drug purchasing time are obtained.

In step S906, a floating window corresponding to the node identifier is generated. The corresponding medical consultation information of the first dimension and medical consultation information of the at least one dimension associated with the medical consultation information of the first dimension are added to the floating window.

Furthermore, a floating window corresponding to the node identifier is generated. The medical consultation information of the first dimension corresponding to the radius where the node identifier is located and medical consultation information of the at least one dimension associated with the medical consultation information of the first dimension are added to the floating window.

In the present embodiment, when obtaining the instruction of viewing node related information, the medical consultation information of various dimensions associated with the node are displayed in the floating window, which makes it easy to view node related information, and further increases the amount of information reflected on the graphics.

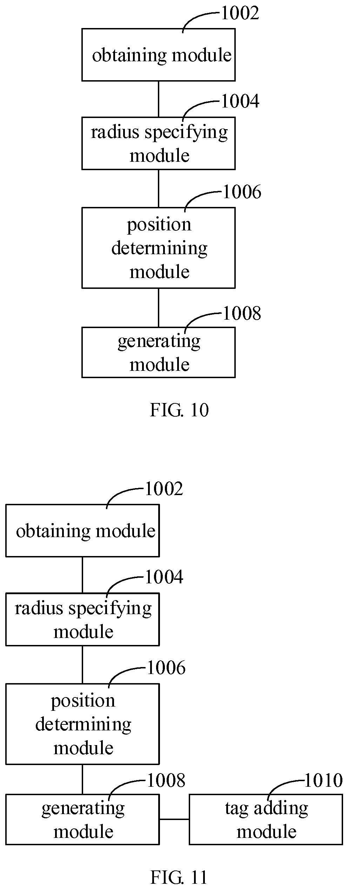

Referring to FIG. 10, a device of generating data is provided. The device includes an obtaining module 1002, a radius forming module 1004, a position determining module 1006, and a generating module 1008.

An obtaining module 1002 configured to obtain data to be processed, and the data to be processed includes drug purchasing time of a time dimension, medical consultation information of at least one dimension besides the time dimension, and association relationship between the medical consultation information of different dimensions. Each dimension includes at least one piece of medical consultation information.

A radius forming module 1004 configured to obtain a preset center point and a preset radius value of a circle, and generate the circle according to the center point and the radius value; select a first dimension from the at least one dimension, and form one corresponding radius in a respective direction in the circle for each piece of the medical consultation information of the first dimension. Each piece of the medical consultation information is corresponding to one radial direction.

A position determining module 1006 configured to obtain the drug purchasing time of the time dimension associated with the medical consultation information of the first dimension according to the association relationship between the medical consultation information of different dimensions, and determine a corresponding position of the associated drug purchasing time of the time dimension on the radius corresponding to the medical consultation information of the first dimension.

A generating module 1008 configured to generate a node corresponding to the associated drug purchasing time of the time dimension at the corresponding position to form a timeline abnormality monitoring graph.

Referring to FIG. 11, in an embodiment, the at least one dimension further includes a second dimension. The obtaining module 1002 is further configured to obtain medical consultation information of the second dimension associated with the medical consultation information of the first dimension within the drug purchasing time of the time dimension associated with the medical consultation information of the first dimension according to the association relationship between the medical consultation information of different dimensions.

In the present embodiment, the device further includes:
A tag adding module 1010 configured to determine corresponding tag information of the node corresponding to the associated drug purchasing time of the time dimension on the radius corresponding to the medical consultation information of the first dimension according to a preset correspondence relationship between the medical consultation information of the second dimension and the tag information, and add the tag information to the corresponding node.

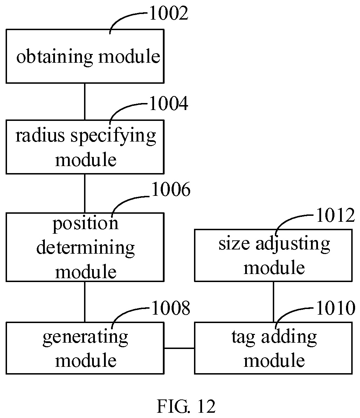

Referring to FIG. 12, in an embodiment, the medical consultation information of the at least one dimension includes a drug purchasing money amount of a money amount dimension. The device further includes:
A size adjusting module 1012 configured to obtain the drug purchasing money amount of the money amount dimension associated with the medical consultation information of the first dimension within the drug purchasing time of the time dimension associated with the medical consultation information of the first dimension according to the association relationship between the medical consultation information of different dimensions, and adjust a size of the node corresponding to the associated drug purchasing time of the time dimension on the radius corresponding to the medical consultation information of the first dimension according to the drug purchasing money amount.

In an embodiment, the position determining module 1006 is further configured to calculate a total time difference between an earliest drug purchasing time and a latest drug purchasing time of the time dimension; calculate a first time sub-difference between the associated drug purchasing time of the time dimension and the earliest drug purchasing time, or calculating a second time sub-difference between the latest drug purchasing time and the associated drug purchasing time of the time dimension; calculate a first ratio of the first time sub-difference to the total time difference, or a second ratio of the second time sub-difference to the total time difference; and determine a position corresponding to the associated drug purchasing time of the time dimension on the radius corresponding to the medical consultation information of the first dimension according to the preset radius value, and the first ratio or the second ratio.

In an embodiment, the radius forming module 1004 is further configured to obtain a preset type corresponding to each piece of the medical information of the first dimension; obtain an angle range of the radius corresponding to each piece of the medical consultation information of the first dimension according to a correspondence relationship between the preset type and the angle range of the radius; and form a radius in a respective direction corresponding to each piece of the medical consultation information of the first dimension, which is corresponding to the angle range, within the obtained angle range in the circle.

In an embodiment, the first dimension includes a drug dimension, the second dimension includes a user dimension, or the first dimension includes a user dimension, the second dimension includes a drug dimension.

The various modules of the device of generating data can be implemented, in part or as a whole, by software, hardware or the combinations thereof. The foregoing modules can be embedded in or independent from a processor of a base station and in the form of hardware, or be stored in a memory of a base station and in the form of software, so as to facilitate the processor to call and execute corresponding steps of the foregoing various modules. The processor can be a central processing unit (CPU), a microprocessor, a micro-controller unit, and so on.

A person skilled in the art should understand that the processes of the methods in the above embodiments can be, in full or in part, implemented by computer programs instructing underlying hardware, the programs can be stored in a computer-readable storage medium, the program can include the processes in the embodiments of the various methods when it is being executed. The storage medium can be a disk, a CD, a Read-Only Memory (ROM) and other non-transitory storage mediums or Random Access Memory (RAM) and so on.

The different technical features of the above embodiments can have various combinations which are not described for the purpose of brevity. Nevertheless, to the extent the combining of the different technical features do not conflict with each other, all such combinations must be regarded as being within the scope of the disclosure.

The foregoing implementations are merely specific embodiments of the present disclosure, and are not intended to limit the protection scope of the present disclosure. It should be noted that any variation or replacement readily figured out by persons skilled in the art within the technical scope disclosed in the present disclosure shall all fall into the protection scope of the present disclosure. Therefore, the protection scope of the present disclosure shall be subject to the protection scope of the claims.

## Claims

1. A method of generating data, comprising:
obtaining data to be processed, wherein the data to be processed comprises a drug purchasing time of a time dimension, medical consultation information of at least one dimension besides the time dimension, and association relationship between the medical consultation information of different dimensions, wherein each dimension comprises at least one piece of medical consultation information;
obtaining a preset center point and a preset radius value of a circle, and generating the circle according to the center point and the radius value; selecting a first dimension from the at least one dimension, and forming one corresponding radius in a respective direction in the circle for each piece of the medical consultation information of the first dimension, wherein each piece of the medical consultation information is corresponding to one radial direction;
obtaining the drug purchasing time of the time dimension associated with the medical consultation information of the first dimension according to the association relationship between the medical consultation information of different dimensions, and determining a corresponding position of the associated drug purchasing time of the time dimension on the radius corresponding to the medical consultation information of the first dimension; and
generating a node corresponding to the associated drug purchasing time of the time dimension at the corresponding position to form a timeline abnormality monitoring graph.

2. The method of claim 1, wherein the at least one dimension further comprises a second dimension;
the method further comprises:
obtaining medical consultation information of the second dimension associated with the medical consultation information of the first dimension within the drug purchasing time of the time dimension associated with the medical consultation information of the first dimension according to the association relationship between the medical consultation information of different dimensions; and
determining corresponding tag information of the node corresponding to the associated drug purchasing time of the time dimension on the radius corresponding to the medical consultation information of the first dimension according to a preset correspondence relationship between the medical consultation information of the second dimension and the tag information, and adding the tag information to the corresponding node.

3. The method of claim 1, wherein the medical consultation information of the at least one dimension comprises a drug purchasing money amount of a money amount dimension;
the method further comprises:
obtaining the drug purchasing money amount of the money amount dimension associated with the medical consultation information of the first dimension within the drug purchasing time of the time dimension associated with the medical consultation information of the first dimension according to the association relationship between the medical consultation information of different dimensions; and
adjusting a size of the node corresponding to the associated drug purchasing time of the time dimension on the radius corresponding to the medical consultation information of the first dimension according to the drug purchasing money amount.

4. The method of claim 1, wherein the determining a corresponding position of the associated drug purchasing time of the time dimension on the radius corresponding to the medical consultation information of the first dimension comprises:
calculating a total time difference between an earliest drug purchasing time and a latest drug purchasing time of the time dimension;
calculating a first time sub-difference between the associated drug purchasing time of the time dimension and the earliest drug purchasing time, or calculating a second time sub-difference between the latest drug purchasing time and the associated drug purchasing time of the time dimension; and
calculating a first ratio of the first time sub-difference to the total time difference, or a second ratio of the second time sub-difference to the total time difference, and determining a position corresponding to the associated drug purchasing time of the time dimension on the radius corresponding to the medical consultation information of the first dimension according to the preset radius value, and the first ratio or the second ratio.

5. The method of claim 1, wherein the forming one corresponding radius in a respective direction in the circle for each piece of the medical consultation information of the first dimension comprises:
obtaining a preset type corresponding to each piece of the medical consultation information of the first dimension;
obtaining an angle range of the radius corresponding to each piece of the medical consultation information of the first dimension according to a correspondence relationship between the preset type and the angle range of the radius; and
forming the radius in a respective direction corresponding to each piece of the medical consultation information of the first dimension, which is corresponding to the angle range, within the obtained angle range in the circle.

6. The method of claim 1, wherein the first dimension comprises a drug dimension, and the second dimension comprises a user dimension; or the first dimension comprises a user dimension, and the second dimension comprises a drug dimension.

7. A device of generating data, comprising:
an obtaining module configured to obtain data to be processed, wherein the data to be processed comprises drug purchasing time of a time dimension, medical consultation information of at least one dimension besides the time dimension, and association relationship between the medical consultation information of different dimensions, wherein each dimension comprises at least one piece of medical consultation information;
a radius forming module configured to obtain a preset center point and a preset radius value of a circle, and generate the circle according to the center point and the radius value; select a first dimension from the at least one dimension, and form one corresponding radius in a respective direction in the circle for each piece of the medical consultation information of the first dimension, wherein each piece of the medical consultation information is corresponding to one radial direction;
a position determining module configured to obtain the drug purchasing time of the time dimension associated with the medical consultation information of the first dimension according to the association relationship between the medical consultation information of different dimensions, and determine a corresponding position of the associated drug purchasing time of the time dimension on the radius corresponding to the medical consultation information of the first dimension; and
a generating module configured to generate a node corresponding to the associated drug purchasing time of the time dimension at the corresponding position to form a timeline abnormality monitoring graph.

8. The device of claim 7, wherein the at least one dimension further comprises a second dimension;
the obtaining module is further configured to obtain medical consultation information of the second dimension associated with the medical consultation information of the first dimension within the drug purchasing time of the time dimension associated with the medical consultation information of the first dimension according to the association relationship between the medical consultation information of different dimensions;
the device further comprises:
a tag adding module configured to determine corresponding tag information of the node corresponding to the associated drug purchasing time of the time dimension on the radius corresponding to the medical consultation information of the first dimension according to a preset correspondence relationship between the medical consultation information of the second dimension and the tag information, and add the tag information to the corresponding node.

9. The device of claim 7, wherein the medical consultation information of the at least one dimension comprises a drug purchasing money amount of a money amount dimension;
the device further comprises:
a size adjusting module configured to obtain the drug purchasing money amount of the money amount dimension associated with the medical consultation information of the first dimension within the drug purchasing time of the time dimension associated with the medical consultation information of the first dimension according to the association relationship between the medical consultation information of different dimensions, and adjust a size of the node corresponding to the associated drug purchasing time of the time dimension on the radius corresponding to the medical consultation information of the first dimension according to the drug purchasing money amount.

10. The device of claim 1, wherein the position determining module is further configured to:
calculate a total time difference between an earliest drug purchasing time and a latest drug purchasing time of the time dimension;
calculate a first time sub-difference between the associated drug purchasing time of the time dimension and the earliest drug purchasing time, or calculating a second time sub-difference between the latest drug purchasing time and the associated drug purchasing time of the time dimension;
calculate a first ratio of the first time sub-difference to the total time difference, or a second ratio of the second time sub-difference to the total time difference; and
determine a position corresponding to the associated drug purchasing time of the time dimension on the radius corresponding to the medical consultation information of the first dimension according to the preset radius value, and the first ratio or the second ratio.

11. The device of claim 7, wherein the radius forming module is further configured to:
obtain a preset type corresponding to each piece of the medical information of the first dimension; obtain an angle range of the radius corresponding to each piece of the medical consultation information of the first dimension according to a correspondence relationship between the preset type and the angle range of the radius; and
form a radius in a respective direction corresponding to each piece of the medical consultation information of the first dimension, which is corresponding to the angle range, within the obtained angle range in the circle.

12. The device of claim 1, wherein the first dimension comprises a drug dimension, and the second dimension comprises a user dimension; or the first dimension comprises a user dimension, and the second dimension comprises a drug dimension.

13. A terminal comprising a processor, and a memory that stores instructions, which, when executed by the processor, cause the processor to perform the following steps:
obtaining data to be processed, wherein the data to be processed comprises a drug purchasing time of a time dimension, medical consultation information of at least one dimension besides the time dimension, and association relationship between the medical consultation information of different dimensions, wherein each dimension comprises at least one piece of medical consultation information;
obtaining a preset center point and a preset radius value of a circle, and generating the circle according to the center point and the radius value; selecting a first dimension from the at least one dimension, and forming one corresponding radius in a respective direction in the circle for each piece of the medical consultation information of the first dimension, wherein each piece of the medical consultation information is corresponding to one radial direction;
obtaining the drug purchasing time of the time dimension associated with the medical consultation information of the first dimension according to the association relationship between the medical consultation information of different dimensions, and determining a corresponding position of the associated drug purchasing time of the time dimension on the radius corresponding to the medical consultation information of the first dimension; and
generating a node corresponding to the associated drug purchasing time of the time dimension at the corresponding position to form a timeline abnormality monitoring graph.

14. The terminal of claim 13, wherein the at least one dimension further comprises a second dimension;
the processor further performs the following steps:
obtaining medical consultation information of the second dimension associated with the medical consultation information of the first dimension within the drug purchasing time of the time dimension associated with the medical consultation information of the first dimension according to the association relationship between the medical consultation information of different dimensions; and
determining corresponding tag information of the node corresponding to the associated drug purchasing time of the time dimension on the radius corresponding to the medical consultation information of the first dimension according to a preset correspondence relationship between the medical consultation information of the second dimension and the tag information, and adding the tag information to the corresponding node.

15. The terminal of claim 13, wherein the medical consultation information of the at least one dimension comprises a drug purchasing money amount of a money amount dimension;
the processor further performs the following steps:
obtaining the drug purchasing money amount of the money amount dimension associated with the medical consultation information of the first dimension within the drug purchasing time of the time dimension associated with the medical consultation information of the first dimension according to the association relationship between the medical consultation information of different dimensions; and
adjusting a size of the node corresponding to the associated drug purchasing time of the time dimension on the radius corresponding to the medical consultation information of the first dimension according to the drug purchasing money amount.

16. The terminal of claim 13, wherein the determining a corresponding position of the associated drug purchasing time of the time dimension on the radius corresponding to the medical consultation information of the first dimension performed by the processor comprises:
calculating a total time difference between an earliest drug purchasing time and a latest drug purchasing time of the time dimension;
calculating a first time sub-difference between the associated drug purchasing time of the time dimension and the earliest drug purchasing time, or calculating a second time sub-difference between the latest drug purchasing time and the associated drug purchasing time of the time dimension; and
calculating a first ratio of the first time sub-difference to the total time difference, or a second ratio of the second time sub-difference to the total time difference, and determining a position corresponding to the associated drug purchasing time of the time dimension on the radius corresponding to the medical consultation information of the first dimension according to the preset radius value, and the first ratio or the second ratio.

17. The terminal of claim 13, wherein the forming one corresponding radius in a respective direction in the circle for each piece of the medical consultation information of the first dimension, performed by the processor comprise:
obtaining a preset type corresponding to each piece of the medical information of the first dimension;
obtaining an angle range of the radius corresponding to each piece of the medical consultation information of the first dimension according to a correspondence relationship between the preset type and the angle range of the radius; and
forming a radius in a respective direction corresponding to each piece of the medical consultation information of the first dimension, which is corresponding to the angle range, within the obtained angle range in the circle.

18. The terminal of claim 13, wherein the first dimension comprises a drug dimension, and the second dimension comprises a user dimension; or the first dimension comprises a user dimension, and the second dimension comprises a drug dimension.

19. A server comprising a processor, and a memory that stores instructions, which, when executed by the processor, cause the processor to perform the following steps:
obtaining data to be processed, wherein the data to be processed comprises a drug purchasing time of a time dimension, medical consultation information of at least one dimension besides the time dimension, and association relationship between the medical consultation information of different dimensions, wherein each dimension comprises at least one piece of medical consultation information;
obtaining a preset center point and a preset radius value of a circle, and generating the circle according to the center point and the radius value; selecting a first dimension from the at least one dimension, and forming one corresponding radius in a respective direction in the circle for each piece of the medical consultation information of the first dimension, wherein each piece of the medical consultation information is corresponding to one radial direction;
obtaining the drug purchasing time of the time dimension associated with the medical consultation information of the first dimension according to the association relationship between the medical consultation information of different dimensions, and determining a corresponding position of the associated drug purchasing time of the time dimension on the radius corresponding to the medical consultation information of the first dimension; and
generating a node corresponding to the associated drug purchasing time of the time dimension at the corresponding position to form a timeline abnormality monitoring graph.

20. The server of claim 19, wherein the at least one dimension further comprises a second dimension;
the processor further performs the following steps:
obtaining medical consultation information of the second dimension associated with the medical consultation information of the first dimension within the drug purchasing time of the time dimension associated with the medical consultation information of the first dimension according to the association relationship between the medical consultation information of different dimensions; and
determining corresponding tag information of the node corresponding to the associated drug purchasing time of the time dimension on the radius corresponding to the medical consultation information of the first dimension according to a preset correspondence relationship between the medical consultation information of the second dimension and the tag information, and adding the tag information to the corresponding node.

21. The server of claim 19, wherein the medical consultation information of the at least one dimension comprises a drug purchasing money amount of a money amount dimension;
the processor further performs the following steps:
obtaining the drug purchasing money amount of the money amount dimension associated with the medical consultation information of the first dimension within the drug purchasing time of the time dimension associated with the medical consultation information of the first dimension according to the association relationship between the medical consultation information of different dimensions; and
adjusting a size of the node corresponding to the associated drug purchasing time of the time dimension on the radius corresponding to the medical consultation information of the first dimension according to the drug purchasing money amount.

22. The server of claim 19, wherein the determining the corresponding position of the associated drug purchasing time of the time dimension on the radius corresponding to the medical consultation information of the first dimension performed by the processor comprising:
calculating a total time difference between an earliest drug purchasing time and a latest drug purchasing time of the time dimension;
calculating a first time sub-difference between the associated drug purchasing time of the time dimension and the earliest drug purchasing time, or calculating a second time sub-difference between the latest drug purchasing time and the associated drug purchasing time of the time dimension; and
calculating a first ratio of the first time sub-difference to the total time difference, or a second ratio of the second time sub-difference to the total time difference, and determining a position corresponding to the associated drug purchasing time of the time dimension on the radius corresponding to the medical consultation information of the first dimension according to the preset radius value, and the first ratio or the second ratio.

23. The server of claim 19, wherein the forming one corresponding radius in a respective direction in the circle for each piece of the medical consultation information of the first dimension performed by the processor comprises:
obtaining a preset type corresponding to each piece of the medical information of the first dimension;
obtaining an angle range of the radius corresponding to each piece of the medical consultation information of the first dimension according to a correspondence relationship between the preset type and the angle range of the radius; and
forming a radius in a respective direction corresponding to each piece of the medical consultation information of the first dimension, which is corresponding to the angle range, within the obtained angle range in the circle.

24. The server of claim 19, wherein the first dimension comprises a drug dimension, and the second dimension comprises a user dimension; or the first dimension comprises a user dimension, and the second dimension comprises a drug dimension.

25. At least one non-transitory computer-readable storage medium that stores computer-executable instructions, which, when executed by at least one processor, cause the at least one processor to perform the following steps:
obtaining data to be processed, wherein the data to be processed comprises a drug purchasing time of a time dimension, medical consultation information of at least one dimension besides the time dimension, and association relationship between the medical consultation information of different dimensions, wherein each dimension comprises at least one piece of medical consultation information;
obtaining a preset center point and a preset radius value of a circle, and generating the circle according to the center point and the radius value; selecting a first dimension from the at least one dimension, and forming one corresponding radius in a respective direction in the circle for each piece of the medical consultation information of the first dimension, wherein each piece of the medical consultation information is corresponding to one radial direction;
obtaining the drug purchasing time of the time dimension associated with the medical consultation information of the first dimension according to the association relationship between the medical consultation information of different dimensions, and determining a corresponding position of the associated drug purchasing time of the time dimension on the radius corresponding to the medical consultation information of the first dimension; and
generating a node corresponding to the associated drug purchasing time of the time dimension at the corresponding position to form a timeline abnormality monitoring graph.
